# EUROPEAN PATENT SPECIFICATION

(11) **EP 1 891 466 B1**
(45) Date of publication and mention of the grant of the patent: **15.09.2010**
(21) Application number: 06772838.6
(22) Date of filing: 12.06.2006
(51) Int. Cl.: G02B 1/04, C08F 290/04, A61L 27/16

(54) **OPHTHALMIC AND OTORHINOLARYNGOLOGICAL DEVICE MATERIALS**
MATERIALIEN FÜR OPHTHALMISCHE UND OTORHINOLARYNGOLOGISCHE GERÄTE
MATERIAUX POUR DISPOSITIFS OPHTALMIQUES ET OTORHINOLARYNGOLOGIQUES

(30) Priority: 13.06.2005 US 689999 P
(43) Date of publication of application: 27.02.2008
(73) Proprietor: ALCON, INC., CH-6331 Hunenberg (CH)
(72) Inventor: SCHLUETER, Douglas, C., Azle, TX 76020 (US)
(74) Representative: Teipel, Stephan
(86) International application number: PCT/US2006/022691
(87) International publication number: WO 2006/138188

(56) References cited:
- WO-A-03/040154
- US-A- 4 085 168
- US-A- 5 693 095
- US-B1- 6 872 793

## Description

### Field of the Invention

This invention is directed to improved ophthalmic and otorhinolaryngological device materials. In particular, this invention relates to soft, high refractive index acrylic device materials that have improved strength.

### Background of the Invention

With the recent advances in small-incision cataract surgery, increased emphasis has been placed on developing soft, foldable materials suitable for use in artificial lenses. In general, these materials fall into one of three categories: hydrogels, silicones, and acrylics.

In general, hydrogel materials have a relatively low refractive index, making them less desirable than other materials because of the thicker lens optic necessary to achieve a given refractive power. Silicone materials generally have a higher refractive index than hydrogels, but tend to unfold explosively after being placed in the eye in a folded position. Explosive unfolding can potentially damage the corneal endothelium and/or rupture the natural lens capsule. Acrylic materials are desirable because they typically have a high refractive index and unfold more slowly or controllably than silicone materials.
U.S. Patent No. 5,290,892 discloses high refractive index, acrylic materials suitable for use as an intraocular lens ("IOL") material. These acrylic materials contain, as principal components, two aryl acrylic monomers. The IOLs made of these acrylic materials can be rolled or folded for insertion through small incisions.
U.S. Patent No. 5,331,073 also discloses soft acrylic IOL materials. These materials contain as principal components, two acrylic monomers which are defined by the properties of their respective homopolymers. The first monomer is defined as one in which its homopolymer has a refractive index of at least about 1.50. The second monomer is defined as one in which its homopolymer has a glass transition temperature less than about 22 °C. These IOL materials also contain a cross-linking component. Additionally, these materials may optionally contain a fourth constituent, different from the first three constituents, which is derived from a hydrophilic monomer. These materials preferably have a total of less than about 15% by weight of a hydrophilic component.
U.S. Patent No. 5,693,095 discloses foldable, high refractive index ophthalmic lens materials containing at least about 90 wt.% of only two principal components: one aryl acrylic hydrophobic monomer and one hydrophilic monomer. The aryl acrylic hydrophobic monomer has the formula 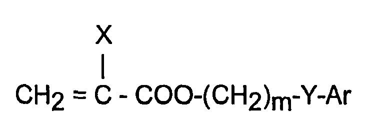 wherein: X is H or CH₃ ;
   m is 0-6;
   Y is nothing, O, S, or NR, wherein R is H, CH₃, CₙH₂ₙ₊₁ (n=1-10), iso-OC₃H₇, C₆H₅, or CH₂C₆H₅; and Ar is any aromatic ring which can be unsubstituted or substituted with CH₃, C₂H₅, n-C₃H₇, iso-C₃H₇, OCH₃, C₆H₁₁, Cl, Br, C₆H₅, or CH₂C₆H₅.
The lens materials described in the '095 Patent preferably have a glass-transition temperature ("Tg") between about -20 and +25 °C.

Flexible intraocular lenses may be folded and inserted through a small incision. In general, a softer material may be deformed to a greater extent so that it can be inserted through an increasingly smaller incision. Soft acrylic or methacrylic materials typically do not have an appropriate combination of strength, flexibility and non-tacky surface properties to permit IOLs to be inserted through an incision as small as that required for silicone IOLs. The mechanical properties of silicone elastomers are improved by addition of an inorganic filler, typically surface treated silica. Surface treated silica improves the mechanical properties of soft acrylic rubbers, too, but reduces the optical clarity of the finished product. Alternative filler materials having a refractive index closer to soft acrylic rubber are needed.

The addition of reinforcing fillers to soft polymers is known to improve tensile strength and tear resistance. Reinforcement stiffens the polymer and improves its toughness by restricting the local freedom of movement of polymer chains, and strengthens the structure by introducing a network of weak fix points. The reinforcing ability of a particular filler depends upon its characteristics (e.g. size and surface chemistry), the type of elastomer with which it is used, and the amount of filler present. Conventional fillers include carbon black and silicate fillers, where the particle size (for maximum surface area) and wettability (for strength of cohesion) are of primary importance. Covalent chemical bonding between the matrix and the filler is generally not required for effective reinforcement. For a recent application and review see: Boonstra, "Role of particulate fillers in elastomer reinforcement: a review" Polymer 1979, 20, 691, and Gu, et al., "Preparation of high strength and optically transparent silicone rubber" Eur. Polym. J. 1998, 34, 1727.

### Summary of the Invention

Improved soft, foldable acrylic device materials which are particularly suited for use as IOLs, but which are also useful as other ophthalmic or otorhinolaryngological devices, such as contact lenses, keratoprostheses, corneal rings or inlays, otological ventilation tubes and nasal implants, have been discovered. These polymeric materials contain microphase-separated domains similar to that found in conventional block copolymers. The presence of the microphase-separated domains improves the strength and influences the surface properties of the polymeric materials without need for added filler materials. The properties of the materials of the present invention are different than statistical (random) copolymers with identical feed ratios.

### Detailed Description of the Invention

Unless indicated otherwise, all component amounts are presented on a % (w/w) basis ("wt.%").

The device materials of the present invention are self-reinforced polymeric materials. The materials are made by the polymerization of a) a monofunctional acrylate or methacrylate monomer [1], b) a difunctional acrylate or methacrylate cross-linker [2], and c) an acrylate or methacrylate terminated polystyrene [3] or a diacrylate or dimethacrylate terminated polystyrene [4]. wherein
R, R' independently = H, CH₃, or CH₂CH₃;
B = O(CH₂)ₙ, NH(CH₂)ₙ, or NCH₃(CH₂)ₙ;
X = O(CH₂)ₙ, NH(CH₂)ₙ, NCH₃(CH₂)ₙ, or nothing;
n=0-6;
Y = phenyl, (CH₂)ₘH, (CH₂)ₘC₆H₅, OH, CH₂CH(OH)CH₂OH, (OCH₂CH₂)ₘOCH₃, or (OCH₂CH₂)ₘOCH₂CH₃;
m=0-12;
Z = (CH₂)ₐ, (CH₂CH₂O)_{b}, O, or nothing;
D = (CH₂)ₐ, O(CH₂CH₂O)_{b}, O, or nothing;
a=1 -12;
b=1-24; and
A = CH₃-, CH₃CH₂-, CH₃CH₂CH₂-, CH₃CH₂CH₂CH₂-, or CH₃CH₂CH(CH₃)-.

Preferred monomers of formula (1) are those wherein:
R = H, B = O(CH₂)₂, Y = phenyl;
R = H, B = O(CH₂)₃, Y = phenyl; and
R = CH₃, B = O(CH₂)₄, Y = phenyl.

Preferred monomers of formula (2) are those wherein:
R = H, X = OCH₂, D = (CH₂)₂
R = CH₃, X = OCH₂, D = nothing; and
R = CH₃, X = nothing, D = O(CH₂CH₂O)_{b}, where b > 10.

Preferred macromers of formula (3) are those wherein:
R = CH₃, R' = H, X = O(CH₂)₂, Z = nothing, A = CH₃CH₂CH(CH₃); and
R = CH₃, R' = CH₃, X = O(CH₂)₂, Z = nothing, A = CH₃CH₂CH(CH₃).

Preferred macromers of formula (4) are those wherein:
R = CH₃, R' = H, X = O(CH₂)₂, Z = nothing, A = CH₃CH₂CH(CH₃); and
R = CH₃, R' = CH₃, X = O(CH₂)₂, Z = nothing, A = CH₃CH₂CH(CH₃).

Monomers of formula (1) are known and can be made by known methods. See, for example, U.S. Patent Nos. 5,331,073 and 5,290,892. Many monomers of formula (1) are commercially available from a variety of sources.

Monomers of formula (2) are known and can be made by known methods, and are commercially available. Preferred monomers of formula (2) include ethylene glycol dimethacrylate; diethylene glycol dimethacrylate; 1,6-hexanediol dimethacrylate; 1,4-butanediol dimethacrylate; poly(ethylene oxide)dimethacrylate (number average molecular weight 600 - 1000); and their corresponding acrylates.

Macromers of formulas (3) and (4) are known. They are commercially available in some instances and can be made by known methods. Macromers of formulas (3) and (4) can be made by covalently attaching a polymerizable group to a functional end group of a linear or branched polystyrene. For example, hydroxyl terminated polystyrene may be synthesized by anionic polymerization of styrene, then functionalized by termination with ethylene oxide to produce hydroxyl terminated polystyrene. The terminal hydroxyl groups are end-capped on one or both terminal chain ends with an acrylate, methacrylate or styrenic group. The end-caps are covalently attached via known methods, for example esterification with methacryloyl chloride or reaction with an isocyanate to form a carbamate linkage. See, generally, U.S. Patent Nos. 3,862,077 and 3,842,059, the entire contents of which are incorporated by reference.

Alternatively, macromers of formula (3) and (4) can also be prepared using atom transfer radical polymerization (ATRP) conditions. For example, a hydroxyl terminal initiator (hydroxyethyl bromoisobutyrate) can combined with copper(I) halide and a solubilizing amine ligand. This can be used to initiate the polymerization of styrene monomer under suitable conditions. See, generally, U.S. Patent Nos. 5,852,129, 5,763,548, and 5,789,487. The resulting hydroxyl terminated poly(styrene) can then be reacted with methacryloyl chloride or isocyanatoethyl methacrylate to produce a methacrylate terminated macromonomer.

The flexibility of the copolymeric material of the present invention depends primarily on the glass transition temperature of the homopolymer formed from monomer (1) and the miscibility of the polystyrene macromer in the resulting polymer network. The concentration of monomer (1) is typically at least 50%, and preferably 65 - 85 wt%, of the total (monomer + macromer + cross-linker) concentration. The difunctional cross-linker (2) concentration is typically 10 to 15 wt% of the total concentration when R = CH₃, X = nothing, D = O(CH₂CH₂O)_{b}, where b > 5 , and preferably less than about 3 wt% for lower molecular weight difunctional cross-linkers, for example when R = H, X = OCH₂, and D = (CH₂)₂. The total concentration of macromers (3) and (4) depends on the glass transition temperature of the homopolymer formed from monomer (1). Macromers (3) and (4) will tend to increase the modulus and decrease the flexibility of the resulting copolymeric material as a function of their molecular weight. At lower molecular weight, the macromers (3) and (4) may be miscible with the resulting polymer network and the effect on T_{g} will be more like a conventional copolymer. At higher molecular weight or higher total macromer concentration, increased phase separation may occur and allow a distinct polystyrene macromer phase and two T_{g}'s. The total concentration of macromers (3) and (4) in the copolymeric material of the present invention typically is between 5 - 40 wt%.

The copolymer clarity is dependent on total macromer concentration and macromer molecular weight in the phenylethylacrylate copolymer series. Phenylethyl acrylate copolymers containing 20 wt% polystyrene macromer with a number average molecular weight (Mₙ) of approximately 51,000 and polydispersity of less than about 1.03 were not optically clear. Copolymers containing lower molecular weight narrow polydispersity polystyrene macromer exhibited excellent optical clarity. Accordingly, the copolymeric materials of the present invention preferably contain macromers (3) or (4) having a Mₙ less than 51,000.

The copolymeric device material of the present invention optionally contains one or more ingredients selected from the group consisting of a polymerizable UV absorber and a polymerizable colorant. Preferably, the device material of the present invention contains no other ingredients besides the monomers of formulas (1) and (2), the macromers (3) and/or (4), and polymerizable UV absorbers and colorants.

The device material of the present invention optionally contains reactive UV absorbers or reactive colorants. A preferred reactive UV absorber is 2-(2'-hydroxy-3'-methallyl-5'-methylphenyl)benzotriazole, commercially available as o-Methallyl Tinuvin P ("oMTP") from Polysciences, Inc., Warrington, Pennsylvania. UV absorbers are typically present in an amount from about 0.1 - 5 % (weight). Suitable reactive blue-light absorbing compounds include those described in U.S. Patent No. 5,470,932. Blue-light absorbers are typically present in an amount from about 0.01 - 0.5 % (weight). When used to make IOLs, the device materials of the present invention preferably contain both a reactive UV absorber and a reactive colorant.

In order to form the device material of the present invention, the chosen ingredients (1), (2), and either (3) or (4) or both (3) and (4) are combined and polymerized using a radical initiator to initiate polymerization by the action of either heat or radiation. The device material is preferably polymerized in de-gassed polypropylene molds under nitrogen or in glass molds.

Suitable polymerization initiators include thermal initiators and photoinitiators. Preferred thermal initiators include peroxy free-radical initiators, such as t-butyl (peroxy-2-ethyl)hexanoate and di-(tert-butylcyclohexyl) peroxydicarbonate (commercially available as Perkadox^{®} 16 from Akzo Chemicals Inc., Chicago, Illinois). Particularly in cases where the materials of the present invention do not contain a blue-light absorbing chromophore, preferred photoinitiators include benzoylphosphine oxide initiators, such as 2,4,6-trimethyl-benzoyldiphenyl-phosphine oxide, commercially available as Lucirin^{®} TPO from BASF Corporation (Charlotte, North Carolina). Initiators are typically present in an amount equal to about 5 % or less of the total formulation weight, and more preferably less than 2 % of the total formulation. As is customary for purposes of calculating component amounts, the initiator weight is not included in the formulation weight % calculation.

The particular combination of the ingredients described above and the identity and amount of any additional components are determined by the desired properties of the finished device material. In a preferred embodiment, the device materials of the present invention are used to make IOLs having an optic diameter of 5.5 or 6 mm that are designed to be compressed or stretched and inserted through surgical incision sizes of 2 mm or less.

The device material preferably has a refractive index in the dry state of at least about 1.47, and more preferably at least about 1.50, as measured by an Abbe' refractometer at 589 nm (Na light source) and 25 °C. Optics made from materials having a refractive index lower than 1.47 are necessarily thicker than optics of the same power which are made from materials having a higher refractive index. As such, IOL optics made from materials with comparable mechanical properties and a refractive index lower than about 1.47 generally require relatively larger incisions for IOL implantation.

The material morphology or phase structure will depend on the macromer concentration, molecular weight, it's miscibility in the copolymer network (which also depends on molecular weight), and the polymerization method. The microphase separated behavior can be observed by differential scanning calorimetry (DSC). Microphase-separated .materials will exhibit two glass-transition temperatures ("T_{g}"). The continuous phase and non-continuous phase will each exhibit a separate T_{g}. T_{g} of the continuous phase will primarily determine the material's flexibility properties, and folding and unfolding characteristics, and is preferably less than about +25 °C, and more preferably less than about 0 °C. T_{g} of the non-continuous phase has a lesser impact on the materials' flexibility than that of the continuous phase. T_{g} is measured by differential scanning calorimetry at 10 °C/min., and is generally determined at the midpoint of the transition of the heat flux versus temperature curve.

The device material preferably has an elongation of at least 150%, more preferably at least 300%, and a Young's modulus of less than 6.0 MPa, more preferably less than 5.0 MPa. These properties indicate that a lens made from such material generally will fold easily and will not crack, tear or split when it is folded. Tensile properties of polymer samples are determined on dumbbell shaped tension test specimens with a 20 mm total length, length in the grip area of 4.88 mm, overall width of 2.49 mm, 0.833 mm width of the narrow section, a fillet radius of 8.83 mm, and a thickness of 0.9 mm. Testing is performed on samples at standard laboratory conditions of 23 ± 2 °C and 50 ± 5% relative humidity using an Instron Material Tester model 4400 with a 50 N load cell. The grip distance is 14 mm and a crosshead speed is 500 mm/minute and the sample is pulled to failure. The elongation (strain) is reported as a fraction of the displacement at failure to the original grip distance ("Elongation" or "Strain at break"). The modulus is calculated as the instantaneous slope of the stress-strain curve at 0% strain ("Young's modulus"), 25% strain ("25% modulus") and 100 % strain ("100% modulus). Tear resistance was measured on unnicked 90 °C angle specimens (Die C) according to ASTM D624-91 "Standard Test Method for Tear Strength of Conventional Vulcanized Rubber and Thermoplastic Elastomers". The test specimens were 20 mm total length, 9.0 mm guage length and a thickness of 0.9 mm. Testing was performed on samples at standard laboratory conditions of 23 ± 2 °C using an Instron Material Tester model 4400 with a 50 N load cell. The grip distance was 9.0 mm and the crosshead speed was 500 mm/minute and the sample was pulled to failure. The tear resistance ("Tear strength") was calculated from the maximum force obtained during testing divided by the sample thickness.

IOLs constructed of the device materials of the present invention can be of any design capable of being stretched or compressed into a small cross section that can fit through a 2-mm incision. For example, the IOLs can be of what is known as a one-piece or multi-piece design, and comprise optic and haptic components. The optic is that portion which serves as the lens and the haptics are attached to the optic and are like arms that hold the optic in its proper place in the eye. The optic and haptic(s) can be of the same or different material. A multi-piece lens is so called because the optic and the haptic(s) are made separately and then the haptics are attached to the optic. In a single piece lens, the optic and the haptics are formed out of one piece of material. Depending on the material, the haptics are then cut, or lathed, out of the material to produce the IOL.

In addition to IOLs, the materials of the present invention are also suitable for use as other ophthalmic or otorhinolaryngological devices such as contact lenses, keratoprostheses, corneal inlays or rings, otological ventilation tubes and nasal implants.

The invention will be further illustrated by the following examples, which are intended to be illustrative, but not limiting.

### Example 1. Thermally initiated copolymerization of methacrylate terminated poly(styrene) with 2-phenylethyl acrylate and 1,4-butanediol diacrylate

A 20-mL scintillation vial was charged with 1.3999 g of methacrylate terminated poly(styrene), 5.6535 g of 2-phenylethyl acrylate (PEA), and 0.0347 g of 1,4-butanediol diacrylate (BDDA). The vial was closed and agitated for about 1 hr to allow the polystyrene component to dissolve. The monomer mixture was filtered through a 1.0-micron glass fiber membrane, then through a 0.45-micron PTFE filter. The formulation was de-gassed by bubbling N₂ through the monomer mixture. t-Butyl peroxy-2-ethylhexanoate (t-BPO) was added (0.0601 g) and the solution was mixed thoroughly. The monomer mixture was dispensed into vacuum de-gassed polypropylene molds under a N₂ atmosphere. The filled molds were then placed in a mechanical convection oven and cured at 70 °C for 1 hr, then post-cured for 2 hrs at 110 °C. The product was removed from the polypropylene molds and the residual monomer was removed by acetone extraction at room temperature. The extracted polymer was dried under vacuum at 60 °C. The percent acetone extractables was determined gravimetrically. Representative properties are listed in Table 1.

### Example 2. Thermally initiated copolymerization of styrene with 2-phenylethyl acrylate and 1,4-butanediol diacrylate

A 20-mL scintillation vial was charged with 2.0096 g of styrene, 7.9588 g of 2-phenylethyl acrylate (PEA), and 0.0565 g of 1,4-butanediol diacrylate (BDDA). The monomer mixture was mixed then filtered through a 0.45-micron PTFE filter. The formulation was de-gassed by bubbling N₂ through the monomer mixture. t-Butyl peroxy-2-ethylhexanoate (t-BPO) was added (0.1050 g) and the solution was mixed throroughly. The monomer mixture was dispensed into vacuum de-gassed polypropylene molds. The filled molds were then placed in a mechanical convection oven and cured at 70 °C for 1 hr, then post-cured for 2 hrs at 110 °C. The product was removed from the polypropylene molds and the residual monomer was removed by acetone extraction at room temperature as indicated in Ex. 1. Representative properties are listed in Table 1.

**Table 1. Comparison of methacrylate terminated poly(styrene) graft copolymer with 2-phenylethyl acrylate and poly(styrene-co-2-phenylethyl acrylate)**

| **Example** | **1** | **2** |
|---|---|---|
| **PEA (wt%)** | 79.76 | 79.39 |
| **Styrene (wt%)** | - | 20.05 |
| **Poly(styrene)MA (wt%)** | 19.74 | - |
| **Poly(styrene)MA Mₙ** | 13,000 | - |
| **%BDDA** | 0.49 | 0.56 |
| **Initiator** | t-BPO | t-BPO |
| **Initiator (wt%)** | 0.85 | 1.05 |
| **Refractive index (25 °C)** | 1.5617 ± 0.0003 | 1.5605 ± 0.0007 |
| **Tensile strength (MPa)** | 8.62 ± 0.79 | 3.81 ± 0.73 |
| **Strain at break (%)** | 957 ± 65 | 783 ± 150 |
| **Young's modulus** | 3.23 ± 0.49 | 6.02 ± 2.14 |
| **25% modulus** | 2.61 ± 0.52 | 6.73 ± 2.08 |
| **100% modulus** | 1.68 ± 0.11 | 2.12 ± 0.49 |
| **Tear resistance (N/mm)** | 6.20 ± 0.87 | 4.54 ± 0.67 |

### Example 3. UV initiated copolymerization of methacrylate terminated poly(styrene) (Mₙ 13,000) with 2-phenylethyl acrylate and 1,4-butanediol diacrylate

A 20-mL scintillation vial was charged with 2.0045 g of methacrylate-terminated polystyrene (Mₙ 13,000), 7.9528 g of 2-phenylethyl acrylate (PEA), and 0.0519 g of 1,4-butanediol diacrylate (BDDA). The vial was closed and the mixture was agitated for about 1 hr to allow the polystyrene component to dissolve. 2-Hydroxy-2-methyl-1-phenylpropane-1-one (Darocur^{®} 1173) was added (0.1050 g) and the solution was mixed thoroughly. The monomer mixture was filtered through a 1.0-micron glass fiber membrane, then a 0.45-micron PTFE membrane filter. The formulation was de-gassed by N₂ bubbling then dispensed into vacuum de-gassed polypropylene molds under a N₂ atmosphere. The filled molds were exposed to UV light for 20 min. The product was removed from the polypropylene molds and the residual monomer was removed by acetone extraction at room temperature as indicated in Ex. 1. Representative properties are listed in Table 2.

### Example 4. UV initiated copolymerization of methacrylate terminated poly(styrene) (Mₙ 23,300) with 2-phenylethyl acrylate and 1,4-butanediol diacrylate

A 20-mL scintillation vial was charged with 0.6002 g of methacrylate-terminated polystyrene (Mₙ 23,300), 2.3937 g of 2-phenylethyl acrylate (PEA), and 0.0172 g of 1,4-butanediol diacrylate (BDDA). The vial was closed and the mixture was agitated for about 1 hr to allow the polystyrene component to dissolve. 2-Hydroxy-2-methyl-1-phenylpropane-1-one (Darocur^{®} 1173) was added (0.0323 g) and the solution was mixed thoroughly. The monomer mixture was filtered through a 1.0-micron glass fiber membrane filter. The formulation was de-gassed by N₂ bubbling then dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 2.

### Example 5. UV initiated copolymerization of methacrylate terminated poly(styrene) (Mₙ 51,000) with 2-phenylethyl acrylate and 1,4-butanediol diacrylate

A 20-mL scintillation vial was charged with 1.0002 g of methacrylate-terminated polystyrene (Mₙ 51,000), 3.9897 g of 2-phenylethyl acrylate (PEA), and 0.0289 g of 1,4-butanediol diacrylate (BDDA). The vial was closed and the mixture was agitated for about 1 hr to allow the polystyrene component to dissolve. 2-Hydroxy-2-methyl-1-phenylpropane-1-one (Darocur^{®} 1173) was added (0.0518 g) and the solution was mixed thoroughly. The monomer mixture was filtered through a 1.0-micron glass fiber membrane filter. The formulation was de-gassed by N₂ bubbling then dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 2.

### Example 6. UV initiated copolymerization of dimethacrylate terminated poly(styrene) with 2-phenylethyl acrylate

A 20-mL scintillation vial was charged with 0.6005 g of dimethacrylate-terminated polystyrene, and 2.4159 g of 2-phenylethyl acrylate (PEA). The vial was closed and the mixture was agitated for about 1 hr to allow the polystyrene component to dissolve. The monomer mixture was filtered through a 1.0-micron glass fiber membrane filter, and de-gassed by N₂ bubbling. 2-Hydroxy-2-methyl-1-phenyl-propane-1-one (Darocur^{®} 1173) was added (0.0285 g) and the solution was mixed thoroughly. The monomer mixture was dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 2.

### Example 7. UV initiated copolymerization of dimethacrylate terminated poly(styrene) with 2-phenylethyl acrylate and 1,4-butanediol diacrylate

A 20-mL scintillation vial was charged with 0.6015 g of dimethacrylate-terminated polystyrene, 2.3960 g of 2-phenylethyl acrylate (PEA), and 0.0164 g of 1,4-butanediol diacrylate (BDDA). The vial was closed and the mixture was agitated for about 1 hr to allow the polystyrene component to dissolve. The monomer mixture was filtered through a 1.0-micron glass fiber membrane filter, and de-gassed by N₂ bubbling. 2-Hydroxy-2-methyl-1-phenyl-propane-1-one (Darocur^{®} 1173) was added (0.0310 g) and the solution was mixed thoroughly. The monomer mixture was dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 2.

**Table 2. Methacrylate and dimethacrylate terminated poly(styrene) copolymers with 2-phenylethyl acrylate and 1,4-butanediol diacrylate**

| **Example** | **3** | **4** | **5** | **6** | **7** |
|---|---|---|---|---|---|
| **PEA (wt%)** | 79.45 | 79.50 | 79.50 | 80.09 | 79.50 |
| **Poly(styrene)MA (wt%)** | 20.03 | 19.93 | 19.93 | - | - |
| **Poly(styrene)MA Mₙ** | 13,000 | 23,300 | 51,000 | - | - |
| **Poly(styrene)DM A (wt%)** | - | -7 | - | 19.91 | 19.96 |
| **Poly(styrene)DM A Mₙ** | - | - | - | 6,200 | 6,200 |
| **%BDDA** | 0.52 | 0.57 | 0.57 | - | 0.54 |
| **Initiator** | Darocur^{®} | Darocur^{®} | Darocur^{®} | Darocur^{®} | Darocur^{®} |
| | 1173 | 1173 | 1173 | 1173 | 1173 |
| **Initiator (wt%)** | 1.02 | 1.07 | 1.03 | 0.94 | 1.03 |
| **Refractive index** | 1.5616 ± | 1.5632 ± | 1.5615 ± | 1.5613 ± | 1.5602 ± |
| **(25 °C)** | 0.0009 | 0.0003 | 0.0005 | 0.0008 | 0.0006 |
| **Tensile strength (MPa)** | 5.62 ± 0.84 | 9.85 ± 0.78 | 933±0.13 | 8.25±0.91 | 7.91 ± 0.93 |
| **Strain at break (%)** | 918 ± 90 | 792 ± 63 | 642 ± 23 | 1084 ± 107 | 774 ± 63 |
| **Young's modulus** | 1.28 ± 0.18 | 2.23 ± 0.21 | 423 ± 0.52 | 4.38 ± 0.52 | 4.93 ± 0.80 |
| **25% modulus** | 1.09 ± 0.25 | 1.52 ± 0.21 | 2.03 ± 0.50 | 4.53 ± 0.45 | 4.91 ± 1.31 |
| **100% modulus** | 0.84 ± 0.03 | 1.46 ± 0.05 | 2.50 ± 0.08 | 2.23 ± 0.16 | 2.57 ± 0.45 |
| **Tear resistance (N/mm)** | 3.99 ± 0.18 | 7.08 ± 1.50 | 6.26 ± 0.98 | 3.85 ± 0.27 | 3.74 ± 0.33 |
| **T_{g} (°C)** | 1 | -1, 90 | -2, 102 | - | - |
| **Appearance** | clear | clear | hazy | clear | clear |

### Example 8. UV initiated copolymerization of methacrylate terminated poly(styrene) with 2-phenylethyl acrylate and 1 wt% 1,4-butanediol diacrylate

A 20-mL scintillation vial was charged with 1.2005 g of methacrylate-terminated polystyrene, 4.7472 g of 2-phenylethyl acrylate (PEA), and 0.0597 g of 1,4-butanediol diacrylate (BDDA). The vial was closed and the mixture was agitated for about 1 hr to allow the polystyrene component to dissolve. The monomer mixture was filtered using a 1.0-micron glass fiber membrane filter, then through a 0.45-micron PTFE membrane filter, and de-gassed by N₂ bubbling. 2-Hydroxy-2-methyl-1-phenyl-propane-1-one (Darocur^{®} 1173) was added (0.0581 g) and the solution was mixed thoroughly. The monomer mixture was dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 3.

### Example 9. UV initiated copolymerization of methacrylate terminated poly(styrene) with 2-phenylethyl acrylate and 2 wt% 1,4-butanediol diacrylate

A 20-mL scintillation vial was charged with 1.2008 g of methacrylate-terminated polystyrene, 4.6929 g of 2-phenylethyl acrylate (PEA), and 0.1225 g of 1,4-butanediol diacrylate (BDDA). The vial was closed and the mixture was agitated for about 1 hr to allow the polystyrene component to dissolve. The monomer mixture was filtered using a 1.0-micron glass fiber membrane filter, then through a 0.45-micron PTFE membrane filter, and de-gassed by N₂ bubbling. 2-Hydroxy-2-methyl-1-phenyl-propane-1-one (Darocur^{®} 1173) was added (0.0561 g) and the solution was mixed thoroughly. The monomer mixture was dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 3.

### Example 10. UV initiated copolymerization of methacrylate terminated poly(styrene) with 2-phenylethyl acrylate and 3 wt% 1,4-butanediol diacrylate

A 20-mL scintillation vial was charged with 1.2008 g of methacrylate-terminated polystyrene, 4.6393 g of 2-phenylethyl acrylate (PEA), and 0.1824 g of 1,4-butanediol diacrylate (BDDA). The vial was closed and the mixture was agitated for about 1 hr to allow the polystyrene component to dissolve. The monomer mixture was filtered using a 1.0-micron glass fiber membrane filter, then through a 0.45-micron PTFE membrane filter, and de-gassed by N₂ bubbling. 2-Hydroxy-2-methyl-1-phenyl-propane-1-one (Darocur^{®} 1173) was added (0.0580 g) and the solution was mixed thoroughly. The monomer mixture was dispensed into vacuum de-gassed polypropylene molds under a N₂ atmosphere. The filled molds were exposed to UV light for 20 min. The product was removed from the polypropylene molds and residual monomer was removed by acetone extraction at room temperature as indicated in Ex. 1. Representative properties are listed in Table 3.

**Table 3. Methacrylate terminated polystyrene copolymers with 2-phenylethyl acrylate and 1,4-butanediol diacrylate**

| **Example** | **8** | **9** | **10** |
|---|---|---|---|
| **PEA (wt%)** | 79.02 | 78.00 | 77.03 |
| **Poly(styrene)MA (wt%)** | 19.98 | 19.96 | 19.94 |
| **Poly(styrene)MA Mₙ BDDA (wt%)** | 13,000 0.99 | 13,000 2.04 | 13,000 3.03 |
| | Darocur^{®} | Darocur^{®} | Darocur^{®} |
| **Initiator** | 1173 | 1173 | 1173 |
| **Initiator (wt%)** | 0.97 | 0.93 | 0.96 |
| **Hydrated clarity (22 °C)** | clear | clear | clear |
| **Hydrated clarity (22 °C)** | haze | slight haze | clear |
| **Tensile strength (MPa)** | 9.27 ± 0.67 | 8.33 ± 0.58 | 7.87 ± 0.38 |
| **Strain at break (%)** | 750 ± 48 | 420 ± 25 | 279 ± 16 |
| **Young's modulus** | 2.74 ± 0.17 | 3.38 ± 0.16 | 4.96 ± 0.62 |
| **25% modulus** | 1.95 ± 0.20 | 2.54 ± 0.43 | 4.18 ± 0.57 |
| **100% modulus** | 1.70 ± 0.09 | 2.37 ± 0.08 | 3.23 ± 0.10 |
| **Refractive index (25 °C)** | 1.5634 0.0002 | ± 1.5631 0.0002 | ± 1.5630 ± 0.0001 |

The addition of polystyrene macromer improves the strength properties of soft acrylic polymers allowing increased distortion without fracture. For example, in Table 1, a 2-phenylethyl acrylate-polystyrene methacrylate graft copolymer (Ex. 1) has increased tensile strength, strain at break, tear resistance, and decreased modulus as compared to a statistical copolymer of 2-phenylethylacrylate and styrene of identical monomer feed ratio. Furthermore, the addition of the styrene component results in an increase in the refractive index as compared to all acrylic formulations, permitting the fabrication of smaller mass lenses of identical refractive power.

The molecular weight of the polystyrene macromer also impacts the polymer properties. In Table 2, Ex. 4 contains a higher Mₙ methacrylate terminated polystyrene, and this results in increased tensile strength and tear resistance with only a moderate increase in modulus as compared to a graft copolymer synthesized with a lower molecular weight methacrylate terminated polystyrene. DSC confirmed the phase-separated morphology in this copolymer (Ex. 4). Further increasing the polystyrene macromer MW resulted in improved tensile properties (Ex. 5), however this copolymer was not optically clear as the phase-separated domains were now large enough to scatter light. Dimethacrylate-terminated polystyrene may also be used with or without additional low molecular weight cross-linker (Table 2, Ex. 6 and 7) to tailor the strain at break.

All of these polymers have excellent clarity as cast. However, the optical clarity following hydration and warming is a function of the cross-linker concentration (Table 3). Copolymers with higher BDDA concentration (Ex. 9, 10) exhibited improved optical clarity when hydrated at 40 °C as compared to lower BDDA concentration (Ex. 8).

### Example 11. Thermally initiated copolymerization of methacrylate terminated poly(styrene) with 2-phenylethyl acrylate, 2-(2-methoxyethoxy)ethyl methacrylate and 1,4-butanediol diacrylate

A 20-mL scintillation vial is charged with 0.80 g of methacrylate terminated poly(styrene), 2.56 g of 2-phenylethyl acrylate (PEA), 0.60 g of 2-(2-methoxyethoxy)ethyl methacrylate (MEEMA), and 0.04 g of 1,4-butanediol diacrylate (BDDA). The vial is closed and agitated to allow the polystyrene macromonomer to dissolve. The monomer mixture is filtered through a 1.0-micron glass fiber membrane. The formulation is de-gassed by bubbling N₂ through the monomer mixture. Di(4-tert-butylcyclohexyl)peroxydicarbonate (Perkadox 16S) is added (0.02 g) and the solution is mixed thoroughly. The monomer mixture is dispensed into vacuum de-gassed polypropylene molds under a N₂ atmosphere. The filled molds are placed in a 70 °C mechanical convection oven for 1 hr, then post-cured at 110 °C for 2 hrs. The product is removed from the polypropylene molds and any residual monomer is removed by acetone extraction at room temperature. The product polymer is dried under vacuum at 60 °C.

These graft copolymers also exhibit a reduced surface tackiness as compared to statistical copolymers of identical feed composition, and this improves the manufacturability and manipulation of IOLs.

This invention has been described by reference to certain preferred embodiments; however, it should be understood that it may be embodied in other specific forms or variations thereof without departing from its special or essential characteristics. The embodiments described above are therefore considered to be illustrative in all respects and not restrictive, the scope of the invention being indicated by the appended claims rather than by the foregoing description.

## Claims

1. A polymeric ophthalmic or otorhinolaryngological device material comprising
a) a monofunctional acrylate or methacrylate monomer [1];
b) a difunctional acrylate or methacrylate cross-linking monomer [2], and
c) an acrylate or methacrylate terminated polystyrene macromer [3] or a diacrylate or dimethacrylate terminated polystyrene macromer [4]. 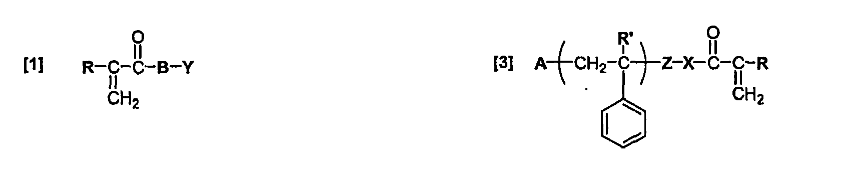 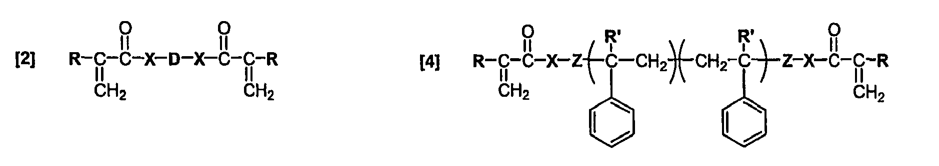 wherein
R, R' independently = H, CH₃, or CH₂CH₃;
B = O(CH₂)ₙ, NH(CH₂)ₙ, or NCH₃(CH₂)ₙ;
X = O(CH₂)ₙ, NH(CH₂)ₙ, NCH₃(CH₂)ₙ, or nothing; n=0-6;
Y = phenyl, (CH₂)ₘH; (CH₂)ₘC₆H₅, OH, CH₂CH(OH)CH₂OH, (OCH₂CH₂)ₘOCH₃, or (OCH₂CH₂)ₘOCH₂CH₃;
m=0-12;
Z = (CH₂)ₐ, (CH₂CH₂O)_{b}, O, or nothing;
D = (CH₂)ₐ, O(CH₂CH₂O)_{b}, O, or nothing;
a=1 -12;
b = 1 - 24; and
A = CH₃-, CH₃CH₂-, CH₃CH₂CH₂-, CH₃CH₂CH₂CH₂-, or
CH₃CH₂CH(CH₃)-.

2. The polymeric device material of Claim 1 wherein the monomer of formula (1) is selected from the group consisting of those wherein
R = H, B = O(CH₂)₂, Y = phenyl; and
R = CH₃, B = O(CH₂)₄, Y = phenyl.

3. The polymeric device material of Claim 1 wherein the monomer of formula (2) is selected from the group consisting of those wherein
R = H, X = OCH₂, D = (CH₂)₂;
R = CH₃, X = OCH₂, D = nothing; and
R = CH₃, X= nothing, D = O(CH₂CH₂O)_{b} where b is > 10.

4. The polymeric device material of Claim 1 wherein the monomer of formula (2) is selected from the group consisting of ethylene glycol dimethacrylate; diethylene glycol dimethacrylate; 1,6-hexanediol dimethacrylate; 1,4-butanediol dimethacrylate; poly(ethylene oxide)dimethacrylate (number average molecular weight 600 - 1000); and their corresponding acrylates.

5. The polymeric device material of Claim 1 wherein the macromer of formula (3) is selected from the group consisting of those wherein
R = CH₃, R' = H, X = O(CH₂)₂, Z = nothing, A = CH₃CH₂CH(CH₃); and
R = CH₃, R' = CH₃, X = O(CH₂)₂, Z = nothing, A = CH₃CH₂CH(CH₃).

6. The polymeric device material of Claim 1 wherein the macromer of formula (4) is selected from the group consisting of those wherein
R = CH₃, R' = H, X = O(CH₂)₂, Z = nothing, A = CH₃CH₂CH(CH₃); and
R = CH₃, R' = CH₃, X = O(CH₂)₂, Z = nothing, A = CH₃CH₂CH(CH₃).

7. The device material of Claim 1 wherein the device material comprises a monomer of formula (1), a monomer of formula (2), a macromer of formula (3), and a macromer of formula (4).

8. The device material of Claim 1 wherein the total amount of monomer of formula (1) is at least 50 % (w/w).

9. The device material of Claim 8 wherein the total amount of monomer of formula (1) is 65 - 85 % (w/w).

10. The device material of Claim 1 wherein the total amount of the monomer of formula (2) is does not exceed 15 % (w/w).

11. The device material of Claim 10 wherein the total amount of monomer of formula (2) is less than 3 % (w/w).

12. The device material of Claim 1 wherein the total amount of macromers (3) and (4) is 5 - 40 % (w/w).

13. The device material of Claim 1 wherein macromer of formula (3) and the macromer of formula (4) have a number average molecular weight less than 51,000.

14. The device material of Claim 1 wherein the device material further comprises an ingredient selected from the group consisting of a polymerizable UV absorber and a polymerizable colorant.

15. The device material of Claim 1 wherein the device material has a refractive index in the dry state of at least 1.47.

16. The device material of Claim 1 wherein the device material has a continuous phase glass transition temperature less than 25 °C.

17. The device material of Claim 1 wherein the device material has an elongation of at least 150% and a Young's modulus of less than 6.0 MPa.

18. An ophthalmic or otorhinolaryngological device comprising the device material of Claim 1 wherein the ophthalmic or otorhinolaryngological device is selected from the group consisting of intraocular lenses; contact lenses; keratoprostheses; corneal inlays or rings; otological ventilation tubes; and nasal implants.

19. An intraocular lens comprising the device material of Claim 1.

## Patentansprüche

1. Polymeres Material für eine ophthalmische oder otorhinolaryngologische Vorrichtung, umfassend
a) ein monofunktionales Acrylat- oder Methacrylatmonomer [1];
b) ein difunktionales Acrylat- oder Methacrylat-Vernetzungsmonomer [2] und
c) ein Acrylat- oder Methacrylat-terminiertes Polystyrolmakromer [3] oder ein Diacrylat- oder Dimethacrylat-terminiertes Polystyrolmakromer [4] 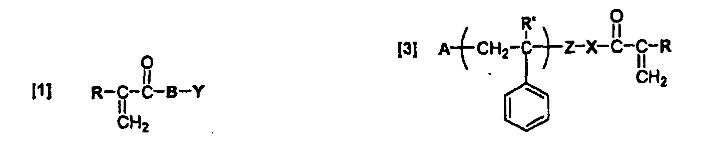 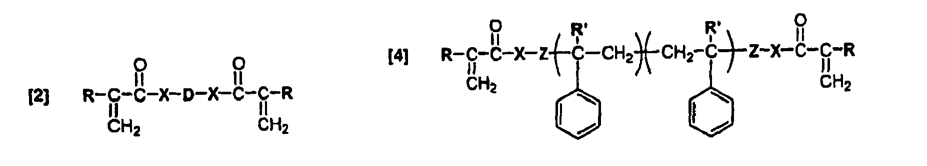 wobei
R, R' unabhängig = H, CH₃ oder CH₂CH₃;
B = O(CH₂)ₙ, NH(CH₂)ₙ oder NCH₃(CH₂)ₙ;
X = O(CH₂)ₙ, NH(CH₂)ₙ, NCH₃(CH₂)ₙ oder nichts;
n=0-6;
Y = Phenyl, (CH₂)ₘH; (CH₂)ₘC₆H₅, OH, CH₂CH(OH)CH₂OH, (OCH₂CH₂)ₘOCH₃ oder (OCH₂CH₂)ₘOCH₂CH₃;
m=0- 12;
Z = (CH₂)ₐ, (CH₂CH₂O)_{b} O oder nichts;
D = (CH₂)ₐ, O(CH₂CH₂O)_{b}, O oder nichts;
a=1-12;
b = 1 - 24 und
A = CH₃-, CH₃CH₂-, CH₃CH₂CH₂-, CH₃CH₂CH₂CH₂- oder CH₃CH₂CH(CH₃)-.

2. Polymeres Material für eine Vorrichtung nach Anspruch 1, wobei das Monomer der Formel (1) ausgewählt ist aus der Gruppe, bestehend aus denen, worin
R = H, B = O(CH₂)₂, Y = Phenyl und
R = CH₃, B = O(CH₂)₄, Y = Phenyl.

3. Polymeres Material für eine Vorrichtung nach Anspruch 1, wobei das Monomer der Formel (2) ausgewählt ist aus der Gruppe, bestehend aus denen, worin
R = H, X = OCH₂, D = (CH₂)₂;
R = CH₃, X = OCH₂, D = nichts und
R = CH₃, X = nichts, D = O(CH₂CH₂O)_{b}, worin b > 10.

4. Polymeres Material für eine Vorrichtung nach Anspruch 1, wobei das Monomer der Formel (2) ausgewählt ist aus der Gruppe, bestehend aus Ethylenglycoldimethacrylat; Diethylenglycoldimethacrylat; 1,6-Hexandioldimethacrylat; 1,4-Butandioldimethacrylat; Poly(ethylenoxid)dimethacrylat (zahlenmittleres Molekulargewicht 600 - 1000) und deren entsprechenden Acrylaten.

5. Polymeres Material für eine Vorrichtung nach Anspruch 1, wobei das Makromer der Formel (3) ausgewählt ist aus der Gruppe, bestehend aus denen, worin
R = CH₃, R' = H, X = O(CH₂)₂, Z = nichts, A = CH₃CH₂CH(CH₃) und
R = CH₃, R' = CH₃, X = O(CH₂)₂, Z = nichts, A = CH₃CH₂CH(CH₃).

6. Polymeres Material für eine Vorrichtung nach Anspruch 1, wobei das Makromer der Formel (4) ausgewählt ist aus der Gruppe, bestehend aus denen, worin
R = CH₃, R' = H, X = O(CH₂)₂, Z = nichts, A = CH₃CH₂CH(CH₃) und
R = CH₃, R' = CH₃, X = O(CH₂)₂, Z = nichts, A = CH₃CH₂CH(CH₃).

7. Material für eine Vorrichtung nach Anspruch 1, wobei das Material für die Vorrichtung ein Monomer der Formel (1), ein Monomer der Formel (2), ein Makromer der Formel (3) und ein Makromer der Formel (4) umfasst.

8. Material für eine Vorrichtung nach Anspruch 1, wobei die Gesamtmenge an Monomer der Formel (1) mindestens 50 % (Gew./Gew.) beträgt.

9. Material für eine Vorrichtung nach Anspruch 8, wobei die Gesamtmenge an Monomer der Formel (1) 65 - 85 % (Gew./Gew.) beträgt.

10. Material für eine Vorrichtung nach Anspruch 1, wobei die Gesamtmenge an Monomer der Formel (2) 15 % (Gew./Gew.) nicht übersteigt.

11. Material für eine Vorrichtung nach Anspruch 10, wobei die Gesamtmenge an Monomer der Formel (2) geringer ist als 3 % (Gew./Gew.).

12. Material für eine Vorrichtung nach Anspruch 1, wobei die Gesamtmenge der Makromere (3) und (4) 5 - 40 % (Gew./Gew.) beträgt.

13. Material für eine Vorrichtung nach Anspruch 1, wobei das Makromer der Formel (3) und das Makromer der Formel (4) ein zahlenmittleres Molekulargewicht von weniger als 51.000 haben.

14. Material für eine Vorrichtung nach Anspruch 1, wobei das Material für die Vorrichtung ferner einen Inhaltsstoff, ausgewählt aus der Gruppe, bestehend aus einem polymerisierbaren UV-Absorber und einem polymerisierbaren Färbemittel, umfasst.

15. Material für eine Vorrichtung nach Anspruch 1, wobei das Material für die Vorrichtung über einen Brechungsindex im Trockenzustand von mindestens 1,47 verfügt.

16. Material für eine Vorrichtung nach Anspruch 1, wobei das Material für die Vorrichtung eine Glasübergangstemperatur in der kontinuierlichen Phase von weniger als 25 °C aufweist.

17. Material für eine Vorrichtung nach Anspruch 1, wobei das Material für die Vorrichtung über eine Dehnung von mindestens 150 % und einen Young'schen Modul von weniger als 6,0 MPa verfügt.

18. Ophthalmische oder otorhinolaryngologische Vorrichtung, umfassend ein Material für eine Vorrichtung nach Anspruch 1, wobei die ophthalmische oder otorhinolaryngologische Vorrichtung ausgewählt ist aus der Gruppe, bestehend aus intraokularen Linsen; Kontaktlinsen; Hornhautprothesen; Hornhautinlays oder -ringen; otologischen Lüftungsschläuchen und Nasenimplantaten.

19. Intraokulare Linse, umfassend das Material für eine Vorrichtung nach Anspruch 1.

## Revendications

1. Matériau polymère pour dispositif ophtalmique ou otorhinolaryngologique, comprenant :
a) un monomère monofonctionnel d'acrylate ou de méthacrylate [1] ;
b) un monomère de réticulation difonctionnel d'acrylate ou de méthacrylate [2], et
c) un macromère de polystyrène à terminaison acrylate ou méthacrylate [3] ou un macromère de polystyrène à terminaison diacrylate ou diméthacrylate [4], 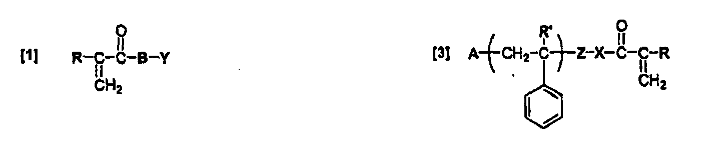 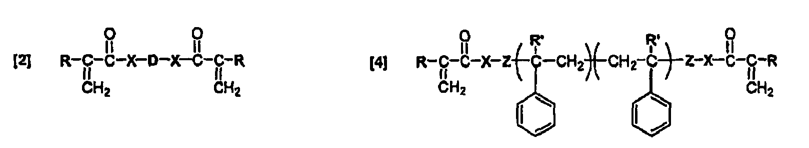 dans lesquels :
R, R' représentent, indépendamment l'un de l'autre, H, CH₃ ou CH₂CH₃ ;
B représente un groupement O (CH₂) ₙ, NH (CH₂) ₙ ou NCH₃(CH₂)ₙ ;
X représente un groupement O(CH₂)ₙ, NH(CH₂)ₙ, NCH₃(CH₂)ₙ ou n'est pas représenté ;
n a une valeur de 0 à 6 ;
Y représente un groupement phényle, (CH₂)ₘH ; (CH₂)ₘC₆H₅, OH, CH₂CH (OH) CH₂OH, (OCH₂CH₂) ₘOCH₃ ou (OCH₂CH₂)ₘOCH₂CH₃ ;
m a une valeur de 0 à 12 ;
Z représente un groupement (CH₂) ₐ, (CH₂CH₂O)_{b}, l'élément O ou n'est pas représenté ;
D représente un groupement (CH₂) ₐ, O(CH₂CH₂O)_{b}, l'élément O ou n'est pas représenté ;
a présente une valeur de 1 à 12 ;
b présente une valeur de 1 à 24 ; et
A représente un groupement CH₃-, CH₃CH₂-, CH₃CH₂CH₂-, CH₃CH₂CH₂CH₂- ou CH₃CH₂CH (CH₃)-.

2. Matériau polymère pour dispositif selon la revendication 1, dans lequel le monomère de formule (1) est choisi dans le groupe constitué de monomères dans lesquels :
R représente H, B représente O(CH₂)₂ et Y représente un phényle ; et
R représente CH₃, B représente O(CH₂)₄, et Y représente un phényle.

3. Matériau polymère pour dispositif selon la revendication 1, dans lequel le monomère de formule (2) est choisi dans le groupe de monomères dans lesquels :
R représente H, X représente OCH₂ et D représente (CH₂)₂ ;
R représente CH₃, X représente OCH₂ et D n'est pas représenté ; et
R représente CH₃, X n'est pas représenté, D représente O(CH₂CH₂O)_{b}, où b > 10.

4. Matériau polymère pour dispositif selon la revendication 1, dans lequel le monomère de formule (2) est choisi dans le groupe constitué du diméthacrylate d'éthylèneglycol ; du diméthacrylate de diéthylèneglycol ; du diméthacrylate de 1,6-hexanediol ; du diméthacrylate de 1,4-butanediol ; du diméthacrylate de poly(oxyde d'éthylène) (poids moléculaire moyen en nombre de 600 à 1000) ; et leurs acrylates correspondants.

5. Matériau polymère pour dispositif selon la revendication 1, dans lequel le monomère de formule (3) est choisi dans le groupe constitué des monomères dans lesquels :
R représente CH₃, R' représente H, X représente O(CH₂)₂, Z n'est pas représenté et A représente CH₃CH₂CH(CH₃) ; et
R représente CH₃, R' représente CH₃, X représente O(CH₂) ₂, Z n'est pas représenté et A représente CH₃CH₂CH(CH₃) .

6. Matériau polymère pour dispositif selon la revendication 1, dans lequel le monomère de formule (4) est choisi dans le groupe constitué des monomères dans lesquels :
R représente CH₃, R' représente H, X représente O(CH₂)₂, Z n'est pas représenté et A représente CH₃CH₂CH (CH₃) ; et
R représente CH₃, R' représente CH₃, X représente O(CH₂)₂, Z n'est pas représenté et A représente CH₃CH₂CH(CH₃).

7. Matériau pour dispositif selon la revendication 1, dans lequel le matériau pour dispositif comprend un monomère de formule (1), un monomère de formule (2), un macromère de formule (3) et un macromère de formule (4).

8. Matériau pour dispositif selon la revendication 1, dans lequel la quantité totale de monomères de formule (1) est d'au moins 50 % (p/p).

9. Matériau pour dispositif selon la revendication 8, dans lequel la quantité totale de monomères de formule (1) est de 65 à 85 % (p/p).

10. Matériau pour dispositif selon la revendication 1, dans lequel la quantité totale de monomères de formule (2) ne dépasse pas 15 % (p/p).

11. Matériau pour dispositif selon la revendication 10, dans lequel la quantité totale de monomères de formule (2) est inférieure à 3 % (p/p).

12. Matériau pour dispositif selon la revendication 1, dans lequel la quantité totale des macromères (3) et (4) est de 5 à 40 % (p/p).

13. Matériau pour dispositif selon la revendication 1, dans lequel le macromère de formule (3) et le macromère de formule (4) ont un poids moléculaire moyen en nombre inférieur à 51 000.

14. Matériau pour dispositif selon la revendication 1, dans lequel le matériau pour dispositif comprend en outre un ingrédient choisi dans le groupe constitué d'un absorbeur d'UV polymérisable et d'un colorant polymérisable.

15. Matériau pour dispositif selon la revendication 1, dans lequel le matériau pour dispositif a un indice de réfraction à l'état sec d'au moins 1,47.

16. Matériau pour dispositif selon la revendication 1, dans lequel le matériau pour dispositif a une température de transition vitreuse de phase continue inférieure à 25 °C.

17. Matériau pour dispositif selon la revendication 1, dans lequel le matériau pour dispositif présente un allongement d'au moins 150 % et un module de Young inférieur à 6,0 MPa.

18. Dispositif ophtalmique ou otorhinolaryngologique comprenant le matériau pour dispositif selon la revendication 1, dans lequel le dispositif ophtalmique ou otorhinolaryngologique est choisi dans le groupe constitué des lentilles intraoculaires ; des lentilles de contact ; des kératoprothèses ; des anneaux cornéens ou des implants intracornéens ; des tubes de ventilation otologiques et des implants nasaux.

19. Lentille intraoculaire comprenant le matériau pour dispositif selon la revendication 1.
